Europäisches Patentamt

European Patent Office

Office européen des brevets

Publication number: **0 197 898**
**B1**

# EUROPEAN PATENT SPECIFICATION

(45) Date of publication of patent specification: **27.06.90**

(21) Application number: **86830083.1**

(22) Date of filing: **07.04.86**

(51) Int. Cl.⁵: **A 61 K 37/12,** A 61 K 9/22 //
(A61K37/12, 31:195)

(54) A formulation based on gelatin and glycine for treating the dryness of the skin.

(30) Priority: **11.04.85 IT 4795085**

(43) Date of publication of application:
**15.10.86 Bulletin 86/42**

(45) Publication of the grant of the patent:
**27.06.90 Bulletin 90/26**

(84) Designated Contracting States:
**BE CH DE FR GB LI LU NL SE**

(56) References cited:
**ROTE LISTE, 1984, Editio Cantor, no. 31467,
Aulendorf/Württ, DE; HERMAL: "Gelacet"**

(73) Proprietor: **Morganti, Pierfrancesco
49, Via Montoggio
I-00168 Roma RM (IT)**

(72) Inventor: **Morganti, Pierfrancesco
49, Via Montoggio
I-00168 Roma RM (IT)**

(74) Representative: **Bazzichelli, Alfredo et al
c/o Società Italiana Brevetti S.p.A. Piazza Poli 42
I-00187 Roma (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates to a formulation based on gelatin and glycine to be administered orally, for the treatment of skin dryness.

The so-called dry skin is the first and the most obvious harm caused by a modification in the moisture content of the epidermis. It occurs more frequently during the cold season, but it can be shown also in other periods of the year, for example following an extended and excessive exposure to sun rays (R. Jackson 27, 106 and B. A. Gilchrest, J. Soc. Cosmet. Chem. 32, 153).

According to researches made by many other authors, the moisture content and state of elasticity of the skin depend essentially on the amount of water present at the level of the horny layer, which is directly linked to a greater or lesser presence of NMF (Natural Moisturising Factor) (J. Blank, J. Invest. Dermatol. 18 433—440 and K. Laden and R. Spitner, J. Soc. Cosmet. Chem. 18, 351).

NMF is a mixture of a series of aminoacids, containing 12% of sodium salt of 5-oxyproline, as well as sugars.

The moisturizing activity effected by NMF would be due essentially to the presence of a glutamic acid derivative, i.e. 5-oxyproline which is more currently known as 2-pyrrolidonecarboxylic acid and indicated by the abbreviation PCA (H. W. Speir and P. G. Pascher, Hautarzt 7, 55, J. D. Middleton, Br. J. Dermat. 80, 437 and S. Tatsumi, Amer. Perf. 87, 61).

PCA, transformed into glutamic acid by 5-oxyprolinase comes to take part together with glycine and cysteine, to the metabolic cycles of glutathione (A. Meister (1983) "Metabolism and transport of glutathione and other γ-glutamyl compounds" in "Functions of glutathione" (A. Larson, S. Orrenius, A. Holmgren and B. Maunervik edits) Raven Press N.Y., page 1).

As it is well-known, glutathione is a tripeptide formed by elementary aminoacids glycine, cysteine and glutamic acid. It is also known that in the metabolic cycle glutathione reaches the interior of the cells, rather than the intercellular tissue, wherein the phenomena inherent to the moisturizing of the skin take place. It is consequently surprising that administering glycine associated with gelatin shows a high favourable effect on the skin moisture, in that, as mentioned above, administration of glutamic acid, to which the presence of NMF is linked, could appear promising to this end from the previous literature.

In the past glycine, which is a known elementary aminoacid, was used for the treatment of muscular distrophy and it is known that an administration thereof orally has a favourable effect on the smooth musculature.

Moreover, it is not known that an administration of gelatin alone could have a favourable influence on cutaneous moisturization.

Gelatin (which in the official pharmacopea is formally denominated "Gelatina F.U." or, according to the International denomination "Gelatinum"), is a known substance which is used, in particular, for the production of medicinal capsules.

In the gelatin itself, among other things, a certain amount of glycine is present and it is a surprising fact that, while gelatin powder has no effect on the cutaneous moisture, the association thereof with glycine, in a determined proportion, makes it possible to obtain the desired effect.

Consequently, the object of the present invention is a formulation for the treatment of skin dryness comprising a mixture of gelatin and glycine in a proportion from 2:1 to 3:1 parts by weight. A proportion of gelatin and glycine of 2,5:1 parts by weight was found particularly effective.

Part of the glycine may be substituted by small quantities of trace elements and vitamins. This addition results in an improvement of the curative effects on dry skin.

In preference, a cutaneous moisturization action has been found on administration of the formulation orally, in the form of soft capsules, formed by a method known as the Scherer method, described for example in the USA patents 2318718, 2356436, 2333433, 2379816, 2378817, 2451141 or also by other methods. Capsules of so-called "soft" gelatin are used as containers for different active pharmaceutical principles. Such capsules of soft gelatin are usally hollow, in that they are intended to contain a medicinal product in the cavity.

It is, however, also possible to use solid spherules. By the term "soft solid gelatin spherules" a spherule free of cavity is intended, wherein the glycine is fused together to the gelatin components, such as to be intimately dispersed therein, rather than contained in a shell of gelatin as a separate phase.

The methods for the preparation of solid spherules are known from the prior art.

As an example, the spherule can weigh 2,8 g., of which 2 g is the amount of gelatin and 800 mg is the amount of glycine.

Experimental tests are referred to hereinbelow.

Example 1
Materials and Methods

Materials

Solid spherules of gelatin/glycine have been used, which had been prepared according to a particular process, each formed by 500 mg gelatin and 200 mg glycine.

Apparatus
Corneometer CM 420

Test of water absorption-ejection

In order to evaluate the moisture condition of the subjects under test measures have been effected in a previously determined area of the right forearm following the method of Tagami and coll. (H. Tagami et al, J. Invest. Dermatol. 78,425).

Said method consists of applying 0,1 ml distilled water on a skin surface of 2,5 cm$^2$. After application (after 10 seconds) the excess water is eliminated by absorbent paper and the first measurement is immediately effected. The measurement is repeated at 30, 60, 90 and 120 seconds.

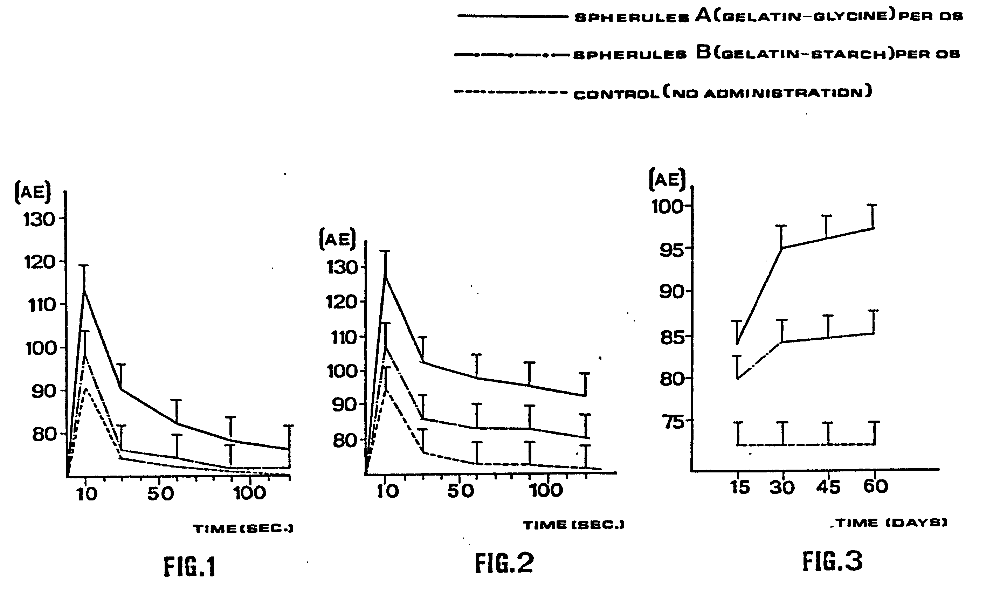

The measurements have been carried out by corneometer CM 420, based on the principle of dielectric constants (K. Mosler, Parf. und Kosmetik 64, 375). The results obtained are referred to in figures 1 and 2.

Evaluation of the cutaneous moisture on human skin

To 30 volunteers of female sex, aged between 30 and 40, all suffering from dry skin, spherules of gelatin-glycine (spherules A) and control spherules (spherules B) wherein glycine was substituted by starch, were delivered following the double blind system. Neither the operator nor the subjects were able to identify the product considered as active.

The spherules were taken orally in a quantity of four a day and for two months, in a period comprised between September and December.

Ten days before and during the treatment the subjects did not use any cosmetic, with the exception of a detergent milk.

Another batch of fifteen control subjects of the same average age made use only of the same detergent milk which was used in the morning as a cleansing milk.

Before beginning the treatment at ten a.m. the condition of moisture and the ability of the skin to bind water (test of absorbtion-ejection) by the subjects, one hour after cleansing was evaluated "in vivo".

The room where all the measures have been carried out was constantly maintained at a constant temperature and moisture ($+18°C$ RH $\geq 50\%$).

Once the test began, readings were taken on the 15th, 30th, 45th and 60th day. On such days, after effecting the direct control reading, the ability of the skin to bind water was also evaluated following the method of water absorbtion-ejection of Tagami. The values as obtained are referred to in table 1 and in a graphic form, in figure 3.

Resuming, in figure 1 are shown the values of water absorbtion ejection after oral administration for 15 days of gelatin-glycine (average of 15 subjects per batch);

in figure 2 are shown the values of the water absorbtion-ejection after an oral administration for 30 days of gelatin/glycine (average of 15 subjects per batch);

in figure 3 is shown the evaluation of the cutaneous moisture after an oral administration of gelatin/glycine (average of 15 subjects per batch); and

in table 1 are shown the values of the evaluation of the cutaneous moisture after an oral administration of gelatin/glycine (average of 5 subjects per batch).

TABLE 1

Evaluation of the cutaneous moisturization after oral administration of
gelatin/glycine (Average of 15 persons per group)

| PATIENT | CONTROLS (no oral administration) days | | | | PATIENT | SPHERULES B (GELATIN/STARCH) days | | | | PATIENT | SPHERULES A (GELATIN/GLYCINE) days | | | |
|---|---|---|---|---|---|---|---|---|---|---|---|---|---|---|
| | 15 | 30 | 45 | 60 | | 15 | 30 | 45 | 60 | | 15 | 30 | 45 | 60 |
| 1 | 73 | 69 | 76 | 75 | 16 | 79 | 85 | 84 | 85 | 31 | 85 | 94 | 94 | 96 |
| 2 | 70 | 74 | 75 | 73 | 17 | 78 | 84 | 82 | 83 | 32 | 87 | 98 | 96 | 99 |
| 3 | 76 | 70 | 77 | 76 | 18 | 83 | 88 | 88 | 87 | 33 | 83 | 92 | 98 | 100 |
| 4 | 75 | 72 | 70 | 77 | 19 | 80 | 87 | 80 | 86 | 34 | 88 | 98 | 96 | 96 |
| 5 | 73 | 77 | 75 | 76 | 20 | 84 | 83 | 84 | 89 | 35 | 82 | 95 | 98 | 100 |
| 6 | 71 | 68 | 73 | 70 | 21 | 82 | 84 | 87 | 88 | 36 | 84 | 98 | 100 | 105 |
| 7 | 70 | 74 | 75 | 73 | 22 | 80 | 85 | 85 | 82 | 37 | 82 | 94 | 98 | 105 |
| 8 | 68 | 73 | 72 | 77 | 23 | 87 | 82 | 83 | 85 | 38 | 84 | 99 | 100 | 99 |
| 9 | 74 | 70 | 75 | 78 | 24 | 82 | 84 | 87 | 82 | 39 | 88 | 98 | 100 | 105 |
| 10 | 69 | 76 | 74 | 72 | 25 | 82 | 86 | 85 | 86 | 40 | 83 | 94 | 96 | 98 |
| 11 | 77 | 71 | 76 | 75 | 26 | 84 | 84 | 87 | 89 | 41 | 87 | 98 | 94 | 96 |
| 12 | 72 | 77 | 70 | 72 | 27 | 81 | 80 | 83 | 84 | 42 | 82 | 93 | 97 | 97 |
| 13 | 70 | 75 | 71 | 76 | 28 | 81 | 80 | 82 | 84 | 43 | 86 | 94 | 99 | 105 |
| 14 | 75 | 68 | 69 | 71 | 29 | 80 | 81 | 83 | 87 | 44 | 86 | 93 | 98 | 100 |
| 15 | 70 | 73 | 77 | 78 | 30 | 81 | 82 | 88 | 85 | 45 | 86 | 98 | 100 | 95 |
| Average | 72,2 | 72,4 | 73,7 | 74,6 | Average | 81 | 83,7 | 84,5 | 85,5 | Average | 84,8 | 95,7 | 97,6 | 99,7 |

EP 0 197 898 B1

Results and comments

As it can be seen from figures 1 and 2, an oral administration of gelatin/glycine considerably increases the ability to bind water by human skin.

Such an increase, which can already be remarked after about a 15 day treatment by the oral pathway, reaches the highest values after 30 days, increasing by 40%. In fact, the values determined on the 60th day do not significantly differ from those evaluated after a 30 day treatment (see figure 3 and table 1).

It will be appreciated that the ability of the skin to retain water can be directly related to the amount of glycine present in the spherules.

Mainly a good improvement was found even in the individuals who had to take product B (control) based on gelatin and starch. As mentioned herein before, gelatin is a protein of animal source, in the aminoacid composition of which about 30% glycine can be found.

The activity performed by gelatin-glycine with respect to the cutaneous moisture can be more clearly seen from figure 3 and table 1.

In fact, a high improvement in the horny layer moisture can be noted, as detected by means of a corneometer CM 4,20. The moisture degree increases by 20% already after the first 30 days of treatment, which successively is maintained to such values during the following days of dietetic therapy.

From figure 3 and table 1 there can be seen also the tendency of gelatin to improve the degree of cutaneous moisture in the treated individuals. Probably glycine through the glutathione cycle increases significantly the production of cellular PCA which can be detected at the level of the horny layer.

## Example 2

The experiment was carried out following the same method and using the same apparatus as described in example 1.

Materials and Methods

Soft gelatin Scherer capsules were used, having the following compositions:

Capsule "A"

| Beeswax | 30 mg |
| Soy lecithin | 34 mg |
| Vegetal oil | 310 mg |
| Starch | 200 mg |
| Gelatin | 250 mg |
| Shell: Gelatin | 186 mg |

Capsule "B"

Starch was replaced by a similar amount of glutamic acid.

Capsule "C"

Starch was replaced by a similar amount of gelatin.

Capsule "D"

Gelatin was replaced by starch and the amount of starch indicated in capsule "A" was replaced by a similar amount of glycine.

Capsule "E"

Starch was replaced by glycine.

Capsule "F"

Starch was replaced by 150 mg glycine additioned with trace elements (ferrous lactate, manganese oxide, copper lactate, tri-calcium phosphate in such an amount as to have 3 mg $Fe^{++}$, 0,5 mg $Mn^{++}$, 0,6 mg $Cu^{++}$, 40 mg $Ca^{++}$) and with vitamines (10 mg Vit. C and 0,4 mg Vit. $B_6$).

Evaluation of the cutaneous moisturization on human skin

Sixty volunteers of female sex divided into 6 lots of 10, aged between 35 and 43, affected by dry skin, were provided with the test products. Each person received randomly from the experimenter the 240 capsules required for the two months of treatment. The capsules, divided into 6 batches, were distinguished with letters A to F, as previously indicated, which are hereinafter briefly resumed:

A) Gelatin + Starch (control)

B) Gelatin + Glutamic acid

C) Gelatin alone
D) Glycine + Starch
E) Gelatin + Glycine
F) Gelatin + trace elements + Vitamines + Glycine
Neither the operator nor the subjects were able to identify the product.
The capsules were administered orally in a dose of four per day and for two months.
The 10 subjects administered with the capsules indicated as "A" have been considered as controls.

Results and Comments
As it can be seen from tables 2, 3, 4 and 5 no positive results have been obtained with the capsules indicated by letters A, B, C and D.
Gelatin alone or additional with glutamic acid, as well as glycine alone,without gelatin, are not capable of improving the moisturization degree of human skin, by the method used.
When gelatin is added to glycine (capsule E) a 12% increase in cutaneous moisturization is obtained after fifteen days only and a 30% increase is obtained after about 30 or 45 days of oral treatment (table 6).
If the amount of glycine is reduced from 200 to 50 mg, however, with an addition of trace elements and specific vitamines (iron, copper, manganese, vitamine C and vitamine $B_6$) a further increase in cutaneous moisturization is verified, namely 30% after 15 days and 40% after about 30 or 45 days of treatment (table 7).

TABLE 2
Capsule "A" (Gelatin + Starch)

|  | CONTROL | | |
| --- | --- | --- | --- |
| PATIENT | 15 | 30 | 45 |
| 1 | 78 | 74 | 76 |
| 2 | 72 | 76 | 78 |
| 3 | 75 | 75 | 78 |
| 4 | 77 | 73 | 75 |
| 5 | 74 | 77 | 73 |
| 6 | 72 | 74 | 76 |
| 7 | 76 | 78 | 73 |
| 8 | 78 | 74 | 72 |
| 9 | 73 | 75 | 77 |
| 10 | 75 | 72 | 76 |
| average | 75.1 | 75.4 | 75.1 |
| variation | 4.09 | 4.44 | 5.69 |
| standard deviation | 2.02 | 2.11 | 2.39 |

TABLE 3
Capsule "B" (Gelatin + Glutamic acid)

| PATIENT | 15 | 30 | 45 |
|---|---|---|---|
| 11 | 78 | 78 | 77 |
| 12 | 75 | 76 | 75 |
| 13 | 73 | 79 | 76 |
| 14 | 75 | 77 | 74 |
| 15 | 76 | 74 | 73 |
| 16 | 74 | 77 | 75 |
| 17 | 78 | 75 | 76 |
| 18 | 76 | 78 | 74 |
| 19 | 73 | 77 | 74 |
| 20 | 73. | 78 | 74 |
| average | 75.1 | 76.9 | 74.8 |
| variation | 3.29 | 2.09 | 1.36 |
| standard deviation | 1.81 | 1.45 | 1.17 |

TABLE 4
Capsule "C" (Gelatin)

| PATIENT | 15 | 30 | 45 |
|---|---|---|---|
| 21 | 76 | 78 | 75 |
| 22 | 73 | 73 | 76 |
| 23 | 74 | 76 | 73 |
| 24 | 75 | 74 | 76 |
| 25 | 78 | 76 | 75 |
| 26 | 72 | 74 | 73 |
| 27 | 72 | 73 | 74 |
| 28 | 73 | 76 | 75 |
| 29 | 75 | 75 | 76 |
| 30 | 77 | 76 | 75 |
| average | 74.5 | 75.1 | 74.8 |
| variation | 3.85 | 2.29 | 1.16 |
| standard deviation | 1.96 | 1.51 | 1.08 |

TABLE 5
Capsule "D" (Glycine + Starch)

| PATIENT | 15 | 30 | 45 |
|---|---|---|---|
| 31 | 74 | 76 | 79 |
| 32 | 77 | 76 | 75 |
| 33 | 73 | 72 | 75 |
| 34 | 78 | 79 | 78 |
| 35 | 75 | 77 | 76 |
| 36 | 72 | 73 | 74 |
| 37 | 76 | 74 | 70 |
| 38 | 75 | 75 | 73 |
| 39 | 78 | 78 | 75 |
| 40 | 73 | 74 | 76 |
| average | 75.1 | 75.4 | 75.1 |
| variation | 4.09 | 4.44 | 5.69 |
| standard deviation | 2.02 | 2.11 | 2.39 |

TABLE 6
Capsule "E" (Gelatin + Glycine)

| PATIENT | 15 | 30 | 45 |
|---|---|---|---|
| 51 | 85 | 99 | 96 |
| 52 | 84 | 97 | 98 |
| 53 | 86 | 89 | 91 |
| 54 | 83 | 92 | 93 |
| 55 | 87 | 97 | 96 |
| 56 | 84 | 98 | 99 |
| 57 | 86 | 95 | 95 |
| 58 | 82 | 93 | 98 |
| 59 | 84 | 90 | 95 |
| 60 | 87 | 97 | 99 |
| average | 84.8 | 94.7 | 96.0 |
| variation | 2.56 | 11.01 | 6.20 |
| standard deviation | 1.60 | 3.32 | 2.49 |

TABLE 7
Capsule "F" (Gelatin + trace elements + Vitamines + Glycine)

| PATIENT | 15 | 30 | 45 |
|---------|------|-------|-------|
| 41 | 91 | 105 | 105 |
| 42 | 89 | 107 | 106 |
| 43 | 90 | 100 | 104 |
| 44 | 92 | 104 | 105 |
| 45 | 94 | 110 | 109 |
| 46 | 92 | 100 | 106 |
| 47 | 94 | 103 | 104 |
| 48 | 96 | 110 | 108 |
| 49 | 90 | 100 | 110 |
| 50 | 93 . | 107 | 110 |
| average | 92.1 | 104.6 | 106.7 |
| variation | 4.29 | 13.64 | 5.01 |
| standard deviation | 2.07 | 3.69 | 2.24 |

A statistical analysis of the results obtained, provides the following comparison table of the theoretical mean values of the cutaneous moisturization degree obtained after administering the different types of capsules.

TABLE 8

THEORETICAL MEAN VALUES

| | 15 days | 30 days | 45 days |
|---|---------|---------|---------|
| CAPSULE A | 74.87 | 75.07 | 75.27 |
| CAPSULE B | 75.75 | 75.60 | 75.45 |
| CAPSULE C | 74.65 | 74.80 | 74.95 |
| CAPSULE D | 75.20 | 75.20 | 75.20 |
| CAPSULE E | 86.10 | 92.10 | 98.10 |
| CAPSULE F | 93.83 | 101.13 | 108.43 |

According to the data obtained in the second experiment, it results that the gelatin used in the capsules or spherules as "carrier" of glycine, is able to make glycine immediately available as an aminoacid necessary for the collagen and probably also elastin formation.

In fact the increase in cutaneous moisturization observed after oral administration of gelatin-glycine, is further increased by 10—15% when glycine is enriched with trace elements $Fe^{++}$, $Cu^{++}$, $Zn^{++}$ and $Ca^{++}$ which are considered as cofactors necessary and required both for the metabolic balance and the building of internal and external bonds of the collagen and elastin molecules with Vitamin C as cofactor in the hydroxylation process of proline and lysine radicals, and with Vitamine $B_6$ as an enzyme (as pyridoxal phosphate) in the oxidative deamination reaction of the free amine group of lysine and oxylysine.

In conclusion from the experimental data and observations, it appears that the combination of gelatin-glycine can act by means of two different biological mechanisms. It promotes the collagen synthesis by raising the deep cutaneous moisturization and activates one or more enzyme systems which are required for a continuous cutaneous production of sodium PCA, which is most responsible for surface moisturizing activity.

**Claims**

1. A pharmaceutical formulation for the treatment of skin dryness, characterised by the fact that it comprises, as an active substance, a composition of soft gelatin and glycine in a proportion from 2:1 to 3:1 parts by weight, wherein up to 0,25 parts of the amount of glycine can be replaced by trace elements and vitamins, as well as a pharmaceutically compatible carrier and excipient.

2. A formulation according to claim 1, wherein the proportion by weight of gelatin and glycine is 2,5:1.

3. Formulation according to claim 1, wherein said trace elements are $Fe^{++}$, $Mn^{++}$, $Cu^{++}$, $Ca^{++}$ and said vitamins are selected from Vitamin C and Vitamin $B_6$.

4. A formulation according to claims 1 and 3, wherein said portion of the amount of glycine which is replaced by trace element ions and vitamins is from 0 to 25% by weight glycine.

5. A formulation according to claims 1 and 3, comprising: 436 g gelatin, 150 g glycine, 3 mg $Fe^{++}$, 0.5 mg $Mn^{++}$, 0.6 mg $Cu^{++}$, 40 mg $Ca^{++}$, 10 mg Vitamin C and 0.4 mg Vitamin $B_6$.

6. Use of a composition comprising gelatin and glycine in a proportion 2:1 to 3:1 by weight, wherein up to 0.25 parts of the amount of glycine can be replaced by trace elements and vitamins, for the manufacture of a medicament for treatment of skin dryness.

**Patentansprüche**

1. Pharmazeutische Formulierung zur Behandlung von Hauttrockenheit, dadurch gekennzeichnet, dass sie als aktiven Bestandteil ein Zusammensetzung aus weicher Gelatine und Glycin in einem Verhältnis 2:1 bis 3:1 Gewichts-%, wobei bis zu 0,25 Teile der Glycinmenge durch Spurelemente und Vitamine ersetzt werden können, sowie pharmazeutisch verträgliche Träger und Exzipienten, enthält.

2. Formulierung gemäss Anspruch 1, wobei das Gewichtsverhältnis der Gelatine zum Glycin 2,5:1 beträgt.

3. Formulierung gemäss Anspruch 1, wobei die Spurelemente $Fe^{++}$, $Mn^{++}$, $Cu^{++}$, $Ca^{++}$ sind, und die Vitamine zwischen Vitamin C und Vitamin $B_6$ ausgewählt werden.

4. Formulierung gemäss den Ansprüchen 1 und 3, wobei der Anteil der Glycinmenge, der durch Spurelementionen und Vitamine ersetzt wird 0 bis 25 Gewichts-% Glycin entspricht.

5. Formulierung gemäss den Ansprüchen 1 und 3, welche 436 g Gelatine, 150 g Glycin, 3 mg $Fe^{++}$, 0,5 mg $Mn^{++}$, 0,6 mg $Cu^{++}$, 40 mg $Ca^{++}$, 10 mg Vitamin C und 0,4 mg Vitamin $B_6$ enthält.

6. Verwendung einer Zusammensetzung, die Gelatine und Glycin in einem Verhältnis von 2:1 bis 3:1 Gewichts-% enthält, wobei bis zu 0,25 Teile der Glycinmenge durch Spurelemente und Vitamine ersetzt werden können, welche zur Herstellung eines Arzneimittels zur Behandlung von Hauttrockenheit dient.

**Revendications**

1. Formulation pharmaceutique pour le traitement de l'aridité de la peau, caractérisé en ce qu'elle comprend, en tant que substance active, une composition de gélatine souple et glycine dans une proportion de 2:1 à 3:1 parts en poids, où jusqu'à 0,25 parties de la quantité de la glycine peut être remplacée par des éléments en trace et des vitamines, ainsi que des véhicules et des excipients pharmaceutiquement compatibles.

2. Formulation selon la revendication 1, où la proportion en poids de gélatine et glycine est de 2.5:1.

3. Formulation pharmaceutique selon la revendication 1, où lesdits éléments en trace sont $Fe^{++}$, $Mn^{++}$, $Cu^{++}$, $Ca^{++}$ et lesdites vitamines sont choisies parmis les vitamine C et vitamine $B_6$.

4. Formulation selon les revendications 1 et 3, où ladite part de la quantité de glycine qui est remplacée par des ions d'éléments en trace et des vitamines est comprise entre 0 et 25% en poids de la glycine.

5. Formulation selon la revendications 1 et 3 comprenant: 436 g de gélatine, 150 g de glycine, 3 mg de $Fe^{++}$, 0,5 mg $Mn^{++}$, 0,6 mg $Cu^{++}$, 40 mg $Ca^{++}$, 10 mg de vitamine C et 0,4 mg de vitamine $B_6$.

6. Utilisation d'une composition comprenant de la gélatine et de la glycine dans une proportion de 2:1 à 3:1 en poids, où lusqu'à 0,25 parts de la quantité de glycine peut être remplacée par des éléments en trace et des vitamines, pour la fabrication d'un médicament pour le traitement de l'aridité de la peau.

SPHERULES A (GELATIN-GLYCINE) PER OS

SPHERULES B (GELATIN-STARCH) PER OS

CONTROL (NO ADMINISTRATION)

FIG.1

FIG.2

FIG.3